# EUROPEAN PATENT APPLICATION

(11) **EP 3 991 791 A1**
(43) Date of publication of application: **04.05.2022**
(21) Application number: 20832977.1
(22) Date of filing: 24.06.2020
(51) Int. Cl.: A61P 3/02, C07C 227/44, C07C 229/36, C07C 277/08, C07C 279/14, C07C 319/26, C07C 323/58, C07D 207/16, A61Q 13/00, A61Q 19/00, A61K 8/44, A23L 2/52, A61K 31/198

(54) **AMINO ACID MIXTURE HAVING CO-AMORPHOUS STRUCTURE**

(30) Priority: 25.06.2019 JP 2019117131
(71) Applicant: AJINOMOTO CO., INC., Chuo-ku Tokyo 104-8315 (JP)
(72) Inventor: SEN Jun, Kawasaki-shi, Kanagawa 210-8681 (JP)
(74) Representative: Strehl Schübel-Hopf & Partner
(86) International application number: PCT/JP2020/024796
(87) International publication number: WO 2020/262455

(57) **Abstract**

The present invention provides a means capable of raising solubility of even a slightly soluble amino acid, without changing to an expensive peptide or varying pH, and it characterized by rendering at least two amino acids to co-amorphous structure.

## Description

### Technical Field

This invention relates to an amino acid mixture having a co-amorphous structure and a method of producing it.

### Background Art

Among foods to which amino acids are supplied, in the case of beverages in a type of dissolving into water to eat it and high-calory solutions for transfusion containing amino acids, various devices have been made in order to dissolve slightly soluble amino acids to a desired concentration.

JP 7-64741 B discloses highly soluble dipeptides containing desired amino acid to be taken, such as L-aspartyl-leucine and L-aspartyl-L-tyrosine. The dipeptides are hydrolyzed into amino acids and absorbed internally.

JP No. 3368897 discloses solutions produced by dissolving 3 types branched chain amino acids composed of isoleucine, leucine and valine into water at a prescribed ratio at pH 4.5 - 2.2 in the presence of an organic acid and/or inorganic acid.

### Prior Art Documents

### Patent Documents

Patent Document 1: JP 7-64741 B
Patent Document 2: JP No. 3368897

### Summary of the Invention

### Problems to be Solved by the Invention

The manufacturing cost of the peptide of the amino acid is higher than the amino acid as is. The method of adjusting pH by an organic acid is sometimes restricted by that it cannot be applied to a product wherein an unstable compound is used in the pH region, or by that only the slightly soluble amino acid is necessary to be dissolved separately.

An object of the invention is to provide a means of raising the solubility of an amino acid without using an expensive peptide or varying pH.

### Means for Solving Problems

The present invention was made in order to solve the above problem, and made by the finding that when two types or more of amino acids are combined and rendered to be amorphous, the co-amorphous structure of both amino acids is kept to be stable.

Namely, the present invention provides an amino acid mixture having a co-amorphous structure, which comprises plural amino acids, wherein at least two amino acids form a co-amorphous structure, the above plural amino acids contains amino acid 1 represented by R₁-CH (NH₂) COOH and amino acid 2 represented by R₂-CH (NH₂) COOH, wherein R₁ and R₂ are selected respectively from the group consisting of hydrogen group, alkyl groups having a carbon number of 1 - 5, and groups containing hydroxyl group, amino group, carboxyl group, aromatic ring, sulfide bond, disulfide bond, thiol group and / or ring structure.

And, the amino acids forming a co-amorphous structure usually consist of the above amino acid 1 and amino acid 2. Therefore, the invention provides an amino acid mixture having a co-amorphous structure, wherein, the amino acid 1 and the amino acid 2 form the co-amorphous structure, the amino acid 1 is represented by R₁-CH (NH₂) COOH and the amino acid 2 is represented by R₂-CH (NH₂) COOH, wherein R₁ and R₂ are selected respectively from the group consisting of hydrogen group, alkyl groups having a carbon number of 1 - 5, and groups containing hydroxyl group, amino group, carboxyl group, aromatic ring, sulfide bond, disulfide bond, thiol group and / or ring structure.

Generally, amino acid crystals have a lattice energy inherent in the material, and have an inherent solubility of the crystal. However, since co-amorphous structure is not crystal, the lattice energy lowers to increase the amount to be dissolved. It was succeeded to increase the amount to be dissolved due to the co-amorphous structure of the amino acid 1 and the amino acid 2, in the invention.

The amino acids employed in the invention exist in general, in a form of crystal. Because the amorphous state of simplex amino acid is unstable, and transfers to crystals, while leaving them. In crystals, molecules are arranged regularly to form crystal lattice. While, in an amorphous state, there is no long distance order, such as crystal lattice, and an interaction which stabilizes solid state, such as lattice energy, is very small. Therefore, molecules disperse and hydrated easily, compared with crystals, and dissolving speed is fast.

Although the present invention is not restrained by any theory, co-amorphous structure is structurally unstable compared with crystals, and it is easily soluble due to its low lattice energy. This is recognized generally in the medical field, where the solubility of crystal is called kinetic solubility and the solubility of amorphous structure is called thermodynamic solubility. Further, the reason why the co-amorphous structure is stable in a supernatant of which amino acid concentration is higher than that of crystals, is that a long time is required for the deposition of the amino acid crystals from the co-amorphous structure compared with crystal. When crystals are dissolved in water, an associate holding crystal structure is formed therein. While, since co-amorphous structure has no crystal structure, completely random associate is formed in water. The random associate is necessary to transfer to associate having crystal structure, and a long time is required for the transfer.

Incidentally, regarding amino acid, solid solutions, e.g. that of three amino acids of valine, leucine and isoleucine are known (WO 2010/050168), and it is also known that the dissolving speed of valine, leucine and isoleucine in the solid solution are improved. However, the solid solution has a structure where a part of the crystal lattice is replaced by another molecule, or where another molecule enters into the space of crystal lattice. Namely, the solid solution is a crystal, and has not a random structure like amorphous structure. The dissolving speed of solid solution is considerably slower than the co-amorphous structure of the invention.

### Effects of the Invention

The amino acid mixture of the invention is highly stable, and raises solubility without using an expensive peptide, and even slightly soluble amino acid, it can be dissolved easily. Moreover, since an amorphous structure in a high energy condition can be maintained stably, there is a merit, such as to raise absorbability of the amino acid or to develop a new use.

### Brief Description of the Drawings

[Figure 1] A drawing of powder X-ray spectra showing that Tyr-Arg is co-amorphous.
[Figure 2] A graph indicating the results of dissolution test of Tyr-Arg co-amorphous material.
[Figure 3] A drawing of powder X-ray spectra obtained in a preservation stability test of Tyr-Arg co-amorphous material.
[Figure 4] A drawing of powder X-ray spectra of Tyr and various amino acids.
[Figure 5] A graph indicating the results of dissolution test of Tyr-LysH co-amorphous material and Tyr crystal.
[Figure 6] A drawing of powder X-ray spectra of Cys2-Arg and simplex Cys2.
[Figure 7] A drawing of powder X-ray spectra of combinations of Cys2 and various amino acids.
[Figure 8] A drawing of powder X-ray spectra of combinations of Leu, etc. and various other amino acids.
[Figure 9] A drawing of powder X-ray spectra of Met-LysH, Met-Arg and simplex Met.
[Figure 10] A drawing of powder X-ray spectra of Tyr-Arg of which pulverization time was changed.
[Figure 11] A drawing of powder X-ray spectra of Tyr-Arg and simplex Tyr produced by a spray dryer.
[Figure 12] A drawing of powder X-ray spectra of Tyr-Arg and Leu-Arg produced by a lyophilizer.

### Mode for Carrying Out the Invention

The amino acids forming the co-amorphous structure of the invention is not limited to α-amino acid, but may be β-amino acid, such as β-alanine, γ-amino acid, such as γ-aminobutyric acid, δ-amino acid or the like.

However, the co-amorphous structure is generally composed of amino acid 1 and amino acid 2. The amino acid 1 is represented by R₁-CH (NH₂) COOH, the amino acid 2 is represented by R₂-CH (NH₂) COOH, and both are α-amino acid. R₁ and R₂ are selected respectively from the group consisting of hydrogen group, alkyl groups having a carbon number 1 - 5, and groups containing at least one of hydroxyl group, amino group, carboxyl group, aromatic ring, sulfide bond, disulfide bond, thiol group and / or ring structure.

The amino acid where R₁ or R₂, is hydrogen group is glycine, and the amino acid where R₁ or R₂ is alkyl group having a carbon number of 1 - 5 is alanine, valine, leucine, isoleucine or the like. The amino acid containing hydroxyl group is serine, threonine or the like, the amino acid containing amino group is lysine, arginine, ornithine, glutamine, asparagine, or the like, and the amino acid containing carboxyl group is aspartic acid, glutamic acid or the like. The amino acid containing aromatic ring is phenylalanine, tyrosine or the like, the amino acid containing sulfide or disulfide bond is methionine, cystine or the like, and the amino acid containing thiol group is cysteine or the like. The amino acid containing ring structure is histidine, tryptophan, proline, hydroxyproline or the like, which contains a heterocyclic ring. The amino acid 1 and amino acid 2 of the invention also include imino acids, such as proline and hydroxyproline.

These amino acids have a degree in tendency to form the co-amorphous structure, and it is preferable that at least one of the amino acids to be combined is liable to form the co-amorphous structure. The amino acids liable to form the co-amorphous structure are basic amino acids containing amino group or imide bond in R₁ or R₂, such as lysine, arginine, ornithine, histidine and their salts, and sulfur-containing amino acids containing sulfide or disulfide bond or thiol group in R₁ or R₂, such as cysteine, cystine and methionine.

An object of the invention is to dissolve slightly soluble amino acids easily, and the slightly soluble amino acids are cystine, tyrosine, phenylalanine, leucine, isoleucine, tryptophan, valine, methionine and the like. The amino acid combined with the slightly soluble amino acid may be any one capable of forming the co-amorphous structure, and is not particularly limited. For example, it may be also a slightly soluble amino acid. Examples of the amino acid being combined with the slightly soluble amino acid are alanine, valine, leucine, isoleucine, lysine, arginine, histidine, ornithine, glutamine, asparagine, aspartic acid, glutamic acid, serine and the like, and preferably, arginine, lysine, histidine and cystine.

Particularly preferable combinations are tyrosine and arginine, tyrosine and serine, tyrosine and lysine hydrochloride, tyrosine and histidine, cystine and arginine, cystine and serine, cystine and sodium glutamate monohydrate, cystine and hydroxyproline, cystine and histidine, cystine and γ-aminobutyric acid, leucine and arginine, leucine and lysine hydrochloride, leucine and histidine, leucine and cystine, isoleucine and arginine, isoleucine and lysine hydrochloride, isoleucine and histidine, isoleucine and cystine, valine and arginine, valine and lysine hydrochloride, valine and histidine, valine and cystine, methionine and arginine, and methionine and lysine hydrochloride.

The combination of the amino acids can be decided by considering its use, and selected from the combinations of amino acids which have been used originally. Moreover, the amino acids are not limited to two types, and three or more amino acids, which form co-amorphous structure, can be combined.

The mixing ratio of the amino acids in the amino acid mixture having a co-amorphous structure of the invention may be any ratio capable of forming the co-amorphous structure, and for example, in the case of two amino acids, the mixing ratio may be 1 : 0.1 - 1 : 10 by molar ratio, preferably 1 : 0.2 - 1 : 5, more preferably 1 : 0.5 - 1 : 2, furthermore preferably 1 : 0.6 - 1 : 2, still more preferably 6 : 4 - 4 : 6, and the most preferably 1 : 1 by molar ratio.

The co-amorphous structure of the invention is a structure where all of two or more amino acids keep a co-amorphous state stably, and in a state where each amino acid is dispersed and mixed uniformly. Every amino acid is in solid, and the degree of dispersion is different according to the preparation. Namely, when a method of dissolving respective amino acids and then solidifying them is utilized, such as spray drying, melt quenching (hot-melt) or lyophilization, each amino acid becomes in a solidified state of solution, namely in a mixed state at a unit of molecule. While, when a method of mixing each amino acid powder as it is and grinding them is utilized, such as grinding by a ball mill, it becomes in a state of mixing fine powders. Even in such a case, it seems that it becomes in a state capable of interacting respective amino acid molecules with each other, and they are rendered to very fine state (about 5 - 30 µm in median diameter).

As to whether each amino acid becomes the co-amorphous structure or not, it can be confirmed by a known method, such as X-ray diffractometry or Raman spectroscopy.

The amino acid mixture of the invention is sufficient where at least two amino acids form into the co-amorphous structure, and three or more amino acids, including the other amino acid(s), may form the co-amorphous structure. In addition, it may include the other amino acid(s) than the at least two amino acids forming into the co-amorphous structure, which does not form the co-amorphous structure, or the other component than amino acid which forms or does not form the co-amorphous structure, or a salt of amino acid or other components.

The co-amorphous structure of amino acid of the invention can be produced by grinding using a ball mill, spray drying, melt quenching, lyophilizing or the like.

As the ball mill, for example, "Ball mill Emax", manufactured by Verder Scientific Co., Ltd. can be used. The method of using the ball mill may be carried out by putting respective amino acids into it at a prescribed mixing ratio, adding a pulverizing assistant, if necessary, and grinding to mix them until becoming into the co-amorphous structure. Each amino acid to be put may be either crystal or amorphous material, and in common, it is crystal. The pulverizing assistant is added in order to prevent concretion, and it is enough that ethanol or the like is added in an amount of about 0.1 - 5 % by weight, preferably 1 - 3 % by weight of total amino acids. Pulverization conditions are to the extent where crystal lattice of each amino acid is collapsed and at least two amino acids form the co-amorphous structure, and may be, generally at a rotary speed of the ball mill of 200 - 1200 rpm for 15 minutes to 12 hours, preferably at 600 - 1200 rpm for 8 - 12 hours, more preferably at 800 - 1200 rpm for 12 hours or more. During pulverizing, since temperature of the ball mill elevates, it is desirable to cool the pot of the ball mill to 15 °C or less, preferably 5 - 12 °C. The pulverizing assistant, such as ethanol, is volatilized while pulverizing, and does not remain in the mixture. As the ball mill, Planetary Ball Mill (manufactured by Kurimoto, Ltd.), Attritor (manufactured by Nippon Coke & Engineering Co., Ltd.), surface modification apparatus (Simoloyer, manufactured by Zoz GmbH) can also be used.

In the case of the production by spray drying, an aqueous solution containing each amino acid at a prescribed ratio is prepared, and it is spray-dried at a temperature, preferably 160 °C or more. It is desirable to evaporate water prior to forming crystal lattice.

The amino acid mixture formed into co-amorphous structure of the invention can be utilized widely for foods and drinks, e.g. drips for supplement, health foods, and moreover, medicines, perfumes and cosmetics, and the like. Particularly, it can be applied to adjusting pH in a state of salt with an organic acid, or substituting for a drip for supplement where an amino acid is made into a dipeptide, such as L-aspartyl-L-leucine or L-aspartyl-L-tyrosine. The amino acids to be combined can be selected from the amino acids employed in each use.

### Examples

### Example 1

### (1) Preparation of Tyr · Arg Co-Amorphous Mixture

5.1 g (0.028 mol) of tyrosine (Tyr) (manufactured by Ajinomoto Co., Inc.), 4.9 g (0.028 mol) of arginine (Arg) (manufactured by Ajinomoto Co., Inc.) and as the pulverizing assistant, 0.1 mL of ethanol were placed in a 125 mL pot made of zirconia. Fifty 10 mm Φ balls made of zirconia were put in the pot to set in a ball mill ("Retsch Emax", manufactured by Verder Scientific Co., Ltd.). The amino acids were ground and mixed at a rotary speed of 1,000 rpm at about 28 °C for 12 hours, while cooling water at 15 °C was streamed into the outer periphery of the pot, and 9.5 g powder was obtained.

### (2) Confirmation Experiment of Forming Amorphous

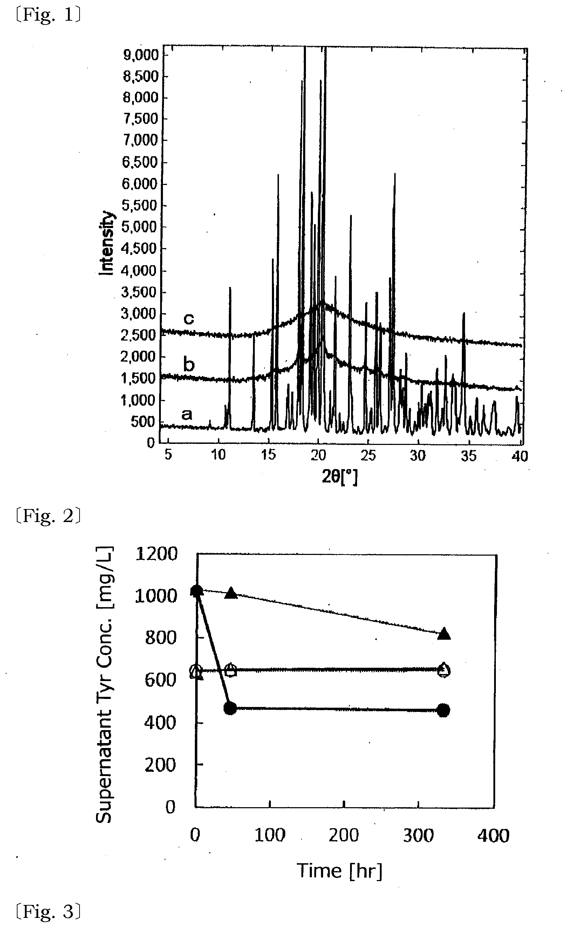

The solid obtained in (1) was measured by the powder X-ray diffraction apparatus ("Empyrean", manufactured by Malvern Panalytical), and the powder X-ray diffraction patterns of the solid were obtained. The results are shown in Figure 1. In the drawing, line a represents the pattern of the mixture at the grinding time of zero hour, line b represents grinding time of 4 hours and line c represents grinding time of 12 hours, respectively. As a result, well-defined peak characteristic of crystal structure was not found. From this, it was confirmed that the solid obtained in (1) was amorphous.

### (3) Dissolved Amount Confirmation Test

Into four 200 mL sample bottles made of glass (A, B, C, D), each 100 mL of ultra-pure water and 138 mg, 138 mg, 220 mg, 220 mg of the solid obtained in (1) in the order from A were put, and stirred by using a magnetic stirrer ("B-1 Magnetic Stirrer Octopus", manufactured by As One Corporation). After the powder was dissolved completely, the pH was adjusted using 5 mol / L hydrochloric acid so as to fall into the range of 6.5 - 7.5. A - D were immersed in a water bath ("NCG-3300", manufactured by Tokyo Rikakikai Co., Ltd.) at 25 °C, A, C were stirred by the magnetic stirrer, and B, D were allowed to stand. After 2 hours, 2 mL was sampled from each sample bottle, and filtered by using the 0.45 µm filter ("Millex", Merck). The filtered sample was diluted 50 times, and Tyr concentration of the supernatant was determined by using the high-performance liquid chromatography (HPLC) ("1100 Series", manufactured by Agilent).

Similar operations were conducted also after 24 hours, 48 hours and 333 hours. The obtained results are shown in Figure 2. In the drawing, ○, △, ● and A represent A (stirred, 138 mg), B (allowed to stand, 138 mg), C (stirred, 220 mg) and D (allowed to stand, 220 mg), respectively. As a result, in the case of A, B, the decrease of supernatant concentration was not found, and to keep 630 mg / L was confirmed. On the other hand, in the case of C, D, the deposition of crystals was recognized, and the decrease of the supernatant Tyr concentration was confirmed. From this, it was confirmed that the state of Tyr concentration of the supernatant of 630 mg / L is kept for 333 hours, irrespective of stirred or allowed to stand conditions (A, B).

### (4) Stability Test

The solid obtained in (1) was placed in an aluminum pouch, further, silica gel was placed, and was preserved in a refrigerator at 4 °C under dehumidified conditions. After 7 days and 16 days, powder X-ray diffraction patterns of the solid were measured by using the powder X-ray diffraction apparatus to obtain the results shown in Figure 3. As a result, well-defined peak characteristic of crystal structure was not found. From this, it was confirmed that the amorphous structure of the solid obtained in (1) was kept for 16 days.

### Examples 2 - 4, Comparative Examples 1 - 6

(1) Preparation of Tyr · Amino Acid 2 Co-Amorphous Mixture Using each amino acid described in Table 1, pulverization of the amino acids was carried out similar to Example 1, except that the pulverization temperature was 30 °C.

**[Table 1]**

| | Amino Acid 1 | | Amino Acid 2 | | Forming Co-Amorphous |
|---|---|---|---|---|---|
| | Type | Weight (g) | Type | Weight (g) | |
| Comparative Ex. 1 | Tyrosine (Tyr) | 10.0 | - | - | × |
| Example 1 | Tyr | 5.1 | Arginine (Arg) | 4.9 | ○ |
| Comparative Ex. 2 | Tyr | 6.1 | Proline (Pro) | 3.9 | × |
| Comparative Ex. 3 | Tyr | 6.7 | Alanine (Ala) | 3.3 | × |
| Comparative Ex. 4 | Tyr | 7.1 | Glycine (Gly) | 2.9 | × |
| Comparative Ex. 5 | Tyr | 6.3 | Hydroxyproline (Hyp) | 4.6 | × |
| Example 2 | Tyr | 6.3 | Serine (Ser) | 3.7 | ○ |
| Comparative Ex. 6 | Tyr | 5.2 | Sodium Glutamate (MSG) | 4.8 | × |
| Example 3 | Tyr | 5.0 | Lysine Hydrochloride (LysH) | 5.0 | ○ |
| Example 4 | Tyr | 5.4 | Histidine (His) | 4.6 | × |

### (2) Confirmation Experiment of Forming Amorphous

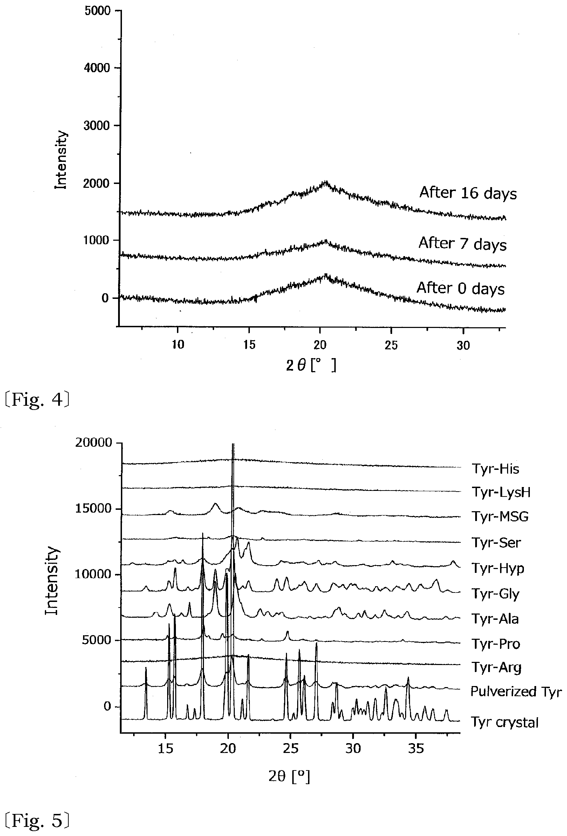

The solid obtained in (1) was measured by the same powder X-ray diffraction apparatus as Example 1, and the powder X-ray diffraction patterns of the solid are shown in Figure 4. From the powder X-ray diffraction patterns, it was confirmed that Tyr-Ser, Tyr-LysH, Tyr-His were formed into co-amorphous. The results of the confirmation are also shown in Table 1.

### (3) Dissolved Amount Confirmation Test

Into two 200 mL sample bottles made of glass (A, B) each 100 mL of ultra-pure water and 130 mg of the solid of Example 3 or Tyr crystal were put, and stirred by using a magnetic stirrer ("B- 1 Magnetic Stirrer Octopus", manufactured by As One Corporation). After the powder was dissolved completely, using 5 mol / L hydrochloric acid, pH was adjusted so as to fall into the range of 6.5 - 7.5. The sample bottles were immersed in a water bath ("NCB-3300", manufactured by Tokyo Rikakikai Co., Ltd.) at 25 °C, and stirred by the magnetic stirrer.

After 5 minutes, 10 minutes, 30 minutes, 1 hour, 2 hours, 24 hours, 168 hours from the start of stirring, 2 mL was sampled from each sample bottle, and filtered by using the 0.45 µm filter ("Millex", Merck). The filtered sample was diluted 50 times, and Tyr concentration of the supernatant was determined by using the high-performance liquid chromatography (HPLC) ("1100 Series", manufactured by Agilent).

The obtained results are shown in Figure 5. It was confirmed that the solid of Example 3 obtained in (1) was dissolved into 600 mg / L after 30 minutes and the concentration was kept for 168 hours, whereas Tyr crystal was dissolved into 430 mg / L after 24 hours.

### Example 5, Comparative Example 7

(1) Preparation of Cys2 · Arg Co-Amorphous Mixture Using 6.76 g (0.0282 mol) of cystine (Cys2) (manufactured by Ajinomoto Co., Inc.) and 3.24 g (0.0186 mol) of arginine (Arg)

(manufactured by Ajinomoto Co., Inc.), pulverization of the amino acids was carried out similar to Example 1, except that the pulverization temperature was 30 °C. In addition, using 10 g of the same cystine, the amino acid was also pulverized similarly.

### (2) Confirmation Experiment of Forming Amorphous

The solid obtained in (1) was measured by the same powder X-ray diffraction apparatus as Example 1, and the powder X-ray diffraction patterns were obtained.

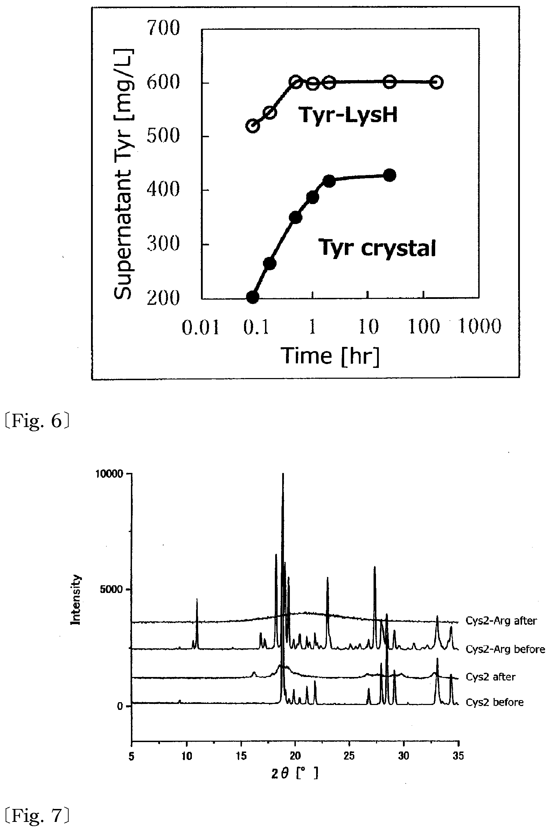

The obtained results are shown in Figure 6. In the drawing, "before" and "after" represent before pulverization and after pulverization, respectively. From the results of Figure 6, well-defined peak characteristic of crystal structure was not found, and it was confirmed that Cys2 · Arg after pulverization was amorphous. On the other hand, in the case of simplex Cys2, crystal property remained.

### Examples 6 - 12

### (1) Preparation of Cys2 · Amino Acid 2 Co-Amorphous Mixture

Using each amino acid described in Table 2, pulverization of the amino acids was carried out similar to Example 1, except that the pulverization temperature was 30 °C, and the pulverizing time was 30 minutes.

**[Table 2]**

| | Amino Acid 1 | | Amino Acid 2 | | Forming Co-Amorphous |
|---|---|---|---|---|---|
| | Type | Weight (g) | Type | Weight (g) | |
| Comparative Ex. 7 | Cystine (Cys2) | 10.0 | - | - | |
| Example 6 | Cys2 | 6.8 | Arg | 3.2 | ○ |
| Example 7 | Cys2 | 7.0 | Ser | 3.0 | ○ |
| Example 8 | Cys2 | 6.2 | MSG | 3.8 | ○ |
| Example 9 | Cys2 | 6.5 | Hypro | 3.5 | ○ |
| Example 10 | Cys2 | 6.1 | His | 3.9 | ○ |
| Example 11 | Cvs2 | 5.7 | LysH | 4.3 | ○ |
| Example 12 | Cys2 | 7.0 | γ-Aminobutyric Acid (GABA) | 3.0 | ○ |

### (2) Confirmation experiment of Forming Amorphous

The solid obtained in (1) was measured by the same powder X-ray diffraction apparatus as Example 1, and the powder X-ray diffraction patterns of the solid are shown in Figure 7. From the powder X-ray diffraction patterns, it was confirmed that Cys2-Arg, Cys2-Ser, Cys2-MSG, Cys2-Hypro, Cys2-His, Cys2-LysH, Cys2-GABA were formed into co-amorphous. The results of the confirmation are also shown in Table 2.

### Examples 13 - 15, Comparative Examples 8 - 11

### (1) Preparation of Amino Acid 1 · Amino Acid 2 Co-Amorphous Mixture

Using each amino acid described in Table 3, pulverization of the amino acids was carried out similar to Example 1, except that the pulverization temperature was 30 °C, and the pulverizing time was 6 hours.

**[Table 3]**

| | Amino Acid 1 | | Amino Acid 2 | | Forming Co-Amorphous |
|---|---|---|---|---|---|
| | Type | Weight (g) | Type | Weight (g) | |
| Comparative Ex. 8 | Leu | 6.4 | Gly | 3.6 | × |
| Comparative Ex. 9 | Leu | 5.6 | Ser | 4.4 | × |
| Comparative Ex. 10 | Leu | 6.0 | Ala | 4.0 | × |
| Example 13 | Leu | 4.3 | Arg | 5.7 | ○ |
| Comparative Ex. 11 | Leu | 5.3 | Pro | 4.7 | × |
| Example 14 | Valine (Val) | 4.0 | Arg | 6.0 | ○ |
| Example 15 | Isoleucine (Ile) | 4.3 | Arg | 5.7 | ○ |

### (2) Confirmation Experiment of Forming Amorphous

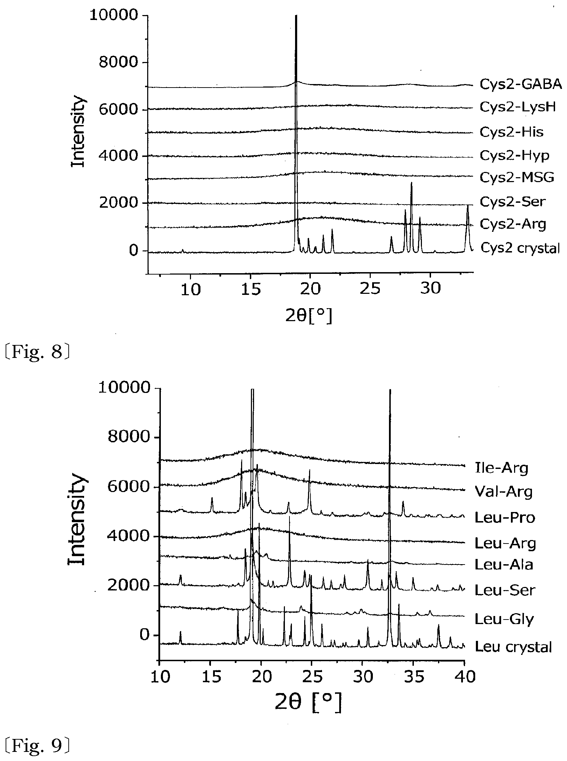

The solid obtained in (1) was measured by the same powder X-ray diffraction apparatus as Example 1, and the powder X-ray diffraction patterns of the solid are shown in Figure 8. From the powder X-ray diffraction patterns, it was confirmed that Leu-Arg, Val-Arg, Ile-Arg were formed into co-amorphous. The results of the confirmation are shown in Table 3.

### Examples 16, 17

### (1) Preparation of Amino acid 1 · Amino Acid 2 Co-Amorphous Mixture

Using each amino acid described in Table 4, pulverization of the amino acids was carried out similar to Example 1, except that the pulverization temperature was 30 °C.

**[Table 4]**

| | Amino Acid 1 | | Amino Acid 2 | | Forming Co-Amorphous |
|---|---|---|---|---|---|
| | Type | Weight (g) | Type | Weight (g) | |
| Example 16 | Methionine (Met) | 4.6 | Arg | 5.4 | ○ |
| Example 17 | Met | 4.5 | LysH | 5.5 | ○ |

### (2) Confirmation Experiment of Forming Amorphous

The solid obtained in (1) was measured by the same powder X-ray diffraction apparatus as Example 1, and the powder X-ray diffraction patterns of the solid are shown in Figure 9. From the powder X-ray diffraction patterns, it was confirmed that Met-Arg, Met-Lys were formed into co-amorphous. The results of the confirmation are shown in Table 4.

### Examples 18-21

As to tyrosine and arginine, pulverization of the amino acids was carried out similar to Example 1, except that the rotary speed was controlled as shown in Table 5. The confirmation results of forming co-amorphous are shown in Table 5.

**[Table 5]**

| | Ecample 18 | Example 19 | Example 20 | Example 21 | Comp. Ex. 11 | Comp. Ex. 12 | Comp. Ex. 13 | Comp. Ex. 14 |
|---|---|---|---|---|---|---|---|---|
| Rotary Speed [rpm] | 1000 | 715 | 650 | 600 | 550 | 500 | 400 | 300 |
| Forming Co-Amorphou s | ○ | ○ | ○ | ○ | × | × | × | × |

### Example 22

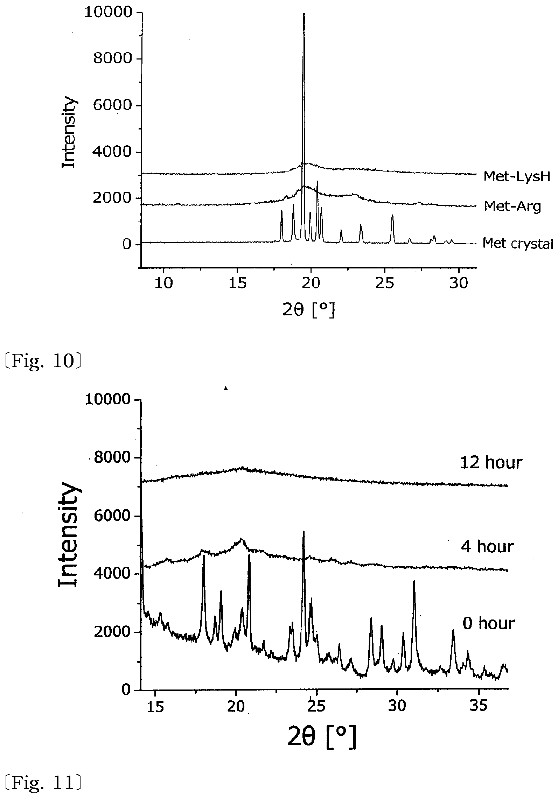

As to tyrosine and arginine, pulverization of the amino acids was carried out similar to Example 1, except that the pulverizing time was controlled as shown in Table 6. The confirmation results of forming co-amorphous are shown in Table 6 and Figure 10.

**[Table 6]**

| | Comparative Ex. 15 | Comparative Ex. 16 | Example 22 |
|---|---|---|---|
| Time [hr] | 0 | 4 | 12 |
| Forming Co-Amorphous | × | × | ○ |

### Example 23 Planetary Ball Mill

20.4 g (0.11 mol) of tyrosine (Tyr) (manufactured by Ajinomoto Co., Inc.), 19.6 g (0.11 mol) of arginine (Arg) (manufactured by Ajinomoto Co., Inc.) and as the pulverizing assistant, 2 mL of ethanol were placed in a 500 mL pot made of zirconia. 456 g of 20 mm Φ balls made of zirconia were put in the pot to set in a planetary ball mill ("BX382", manufactured by Kurimoto Ltd.). Nitrogen gas was enclosed in the pot, and grinding and mixing were carried out for 5 hours at the revolution speed of 382 rpm and the rotation speed of 842 rpm to obtain 37.7 g of co-amorphous powder. While grinding, the temperature was raised from room temperature to 60 °C at maximum.

### Example 24 Attoritor

143 g (0.79 mol) of tyrosine (Tyr) (manufactured by Ajinomoto Co., Inc.), 137 g (0.79 mol) of arginine (Arg) (manufactured by Ajinomoto Co., Inc.) and as the pulverizing assistant, 4 mL of ethanol were placed in a vessel made of stainless steel. 11.9 kg of 15 mm Φ balls made of zirconia, and set in an attoritor ("MAID", manufactured by Nippon Coke & Engineering Co., Ltd.). Nitrogen gas was enclosed in the vessel, and grinding and mixing were carried out for 4 hours at the revolution speed of the arm of 300 rpm, while tap water was streamed into the outer periphery of the vessel, to obtain 250g of co-amorphous material. Example 25 Surface Modification Apparatus (Simoloyer)

102 g (0.56 mol) of Tyrosine (Tyr) (manufactured by Ajinomoto Co., Inc.), 98 g (0.56 mol) of arginine (Arg) (manufactured by Ajinomoto Co., Inc.), and 4 mL of ethanol as the pulverizing assistant were placed in a 2 L vessel made of alumina. 2 kg of 5 mm Φ balls made of zirconia were put in the pot to set in a surface modification apparatus ("CM-01", manufactured by Zoz GmbH). Grinding and mixing were carried out for 3 hours at a revolution speed of the arm of 1000 rpm, while cooling water at 10 °C was streamed into the outer periphery of the pot, to obtain 184 g of co-amorphous powder.

### Example 26 Spray Dryer

3.60 g of tyrosine and 3.46 g of arginine were put into 12 L of water to dissolve them. Using a spray dryer ("GB210", manufactured by Yamato Scientific Co., Ltd.), the solution of tyrosine and arginine was spray-dried at the air flow of 0.51 m³ / min, the inlet temperature of 180 - 190 °C, the outlet temperature of 60 °C, the pressure of the atomizer gas of 0.13 MPa, liquid feeding rate of 20 mL / min to obtain 520 mg of powder. It was confirmed to form co-amorphous by the powder X-ray diffraction spectrum. The powder X-ray diffraction patterns are shown in Figure 11.

### Example 27

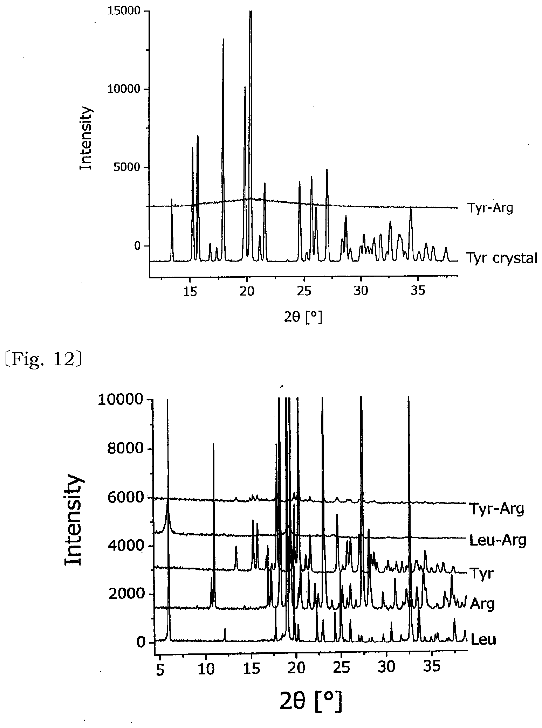

500 mg of leucine and 664 mg of arginine were dissolved in 100 mL of water, and frozen in a freezer at -80 °C. Using the lyophilizer ("FDU-1200", manufactured by Tokyo Rikakikai Co., Ltd.), vacuum crying was conducted at a temperature of -45 °C, vacuum degree of 21 Pa for 4 days, to obtain 1013 mg of powder (yield: 87 %). The powder X-ray diffraction patterns are sown in Figure 12. From the powder X-ray diffraction analysis, it appears that co-amorphous formation progressed, but did not become complete co-amorphous material.

### Example 28

300 mg of tyrosine and 288 mg of arginine were dissolved in 100 mL of water, and frozen in a freezer at -80 °C. Using the lyophilizer ("FDU-1200", manufactured by Tokyo Rikakikai Co., Ltd.), vacuum drying was conducted at a temperature of -45 °C, vacuum degree of 21 Pa for 4 days, to obtain 419 mg of powder (yield: 71 %). The powder X-ray diffraction patterns are shown in Figure 12. From the powder X-ray diffraction analysis, it appears that co-amorphous formation progressed, but did not become complete co-amorphous material.

### Industrial Applicability

The present invention can be utilized widely in the fields of various culture medium, food, medicine, etc. where 2 or more amino acids are used, and is particularly effective for the use containing dissolution of slightly soluble amino acid.

## Claims

1. An amino acid mixture having a co-amorphous structure, wherein at least amino acid 1 and amino acid 2 are formed into the co-amorphous structure among plural amino acids, said amino acid 1 is represented by R₁-CH (NH₂) COOH and said amino acid 2 is represented by R₂-CH (NH₂) COOH, wherein R₁ and R₂ are selected respectively from the group consisting of hydrogen group, alkyl groups having a carbon number of 1 - 5, and groups containing hydroxyl group, amino group, carboxyl group, aromatic ring, sulfide bond, disulfide bond, thiol group and / or ring structure.

2. An amino acid mixture having a co-amorphous structure, wherein amino acid 1 and amino acid 2 are formed into the co-amorphous structure, said amino acid 1 is represented by R₁-CH (NH₂) COOH and said amino acid 2 is represented by R₂-CH (NH₂) COOH, wherein R₁ and R₂ are selected respectively from the group consisting of hydrogen group, alkyl groups having a carbon number of 1 - 5, and groups containing hydroxyl group, amino group, carboxyl group, aromatic ring, sulfide bond, disulfide bond, thiol group and / or ring structure.

3. The amino acid mixture as set forth in claim 1 or 2, wherein at least one of said R1 and R2 contains at least one of guanidyl group, imidazoyl group, 4-aminobutyl group, S-methyl thioether group, disulfide bond or thiol group.

4. The amino acid mixture as set forth in any one of claims 1 - 3, wherein either said amino acid 1 or amino acid 2 is an amino acid selected from lysine, arginine, histidine, cystine, methionine, cysteine or a salt thereof.

5. The amino acid mixture as set forth in any one of claims 1 - 4, wherein either said amino acid 1 or amino acid 2 is an amino acid selected from cystine, tyrosine, phenylalanine, leucine, isoleucine or tryptophan.

6. The amino acid mixture as set forth in any one of claims 1 - 5, wherein the amino acids which constitute the co-amorphous structure are a combination of tyrosine and arginine, tyrosine and serine, tyrosine and lysine hydrochloride, tyrosine and histidine, cystine and arginine, cystine and serine, cystine and sodium glutamate monohydrate, cystine and hydroxyproline, cystine and histidine, cystine and γ-aminobutyric acid, leucine and arginine, leucine and lysine hydrochloride, leucine and histidine, leucine and cystine, isoleucine and arginine, isoleucine and lysine hydrochloride, isoleucine and histidine, isoleucine and cystine, valine and arginine, valine and lysine hydrochloride, valine and histidine, valine and cystine, methionine and arginine, methionine and lysine hydrochloride.

7. The amino acid mixture as set forth in any one of claims 1 - 6, wherein the mixing ratio of amino acid 1 to amino acid 2 in the amino acid mixture having a co-amorphous structure is 0.1 - 10 by molar ratio.

8. The amino acid mixture as set forth in claims 7, wherein the mixing ratio of amino acid 1 to amino acid 2 is 6 : 4 - 4 : 4 by molar ratio.

9. A beverage containing the amino acid mixture as set forth in any one of claims 1-8.

10. A medicine containing the amino acid mixture as set forth in any one of claims 1-8.

11. An aromatic or cosmetic containing the amino acid mixture as set forth in any one of claims 1-8.

12. A method of producing the amino acid mixture as set forth in any one of claims 1-8, which comprises purring at least two amino acids among plural amino acid into a ball mill at a molar ratio of 1 : 0.1-10, and also adding a pulverizing assistant at 0.1 - 5 weight % of total amount of total amino acids, and driving the ball mill at a rotational speed of 200 - 1200 rpm for 15 minutes to 12 hours.

13. A method of producing the amino acid mixture as set forth in any one of claims 12, wherein the pot of the ball mill is cooled to 5 - 15 °C while driving the ball mill.
